# EUROPEAN PATENT APPLICATION

(11) **EP 4 684 818 A1**
(43) Date of publication of application: **28.01.2026**
(21) Application number: 25188561.2
(22) Date of filing: 09.07.2025
(51) Int. Cl.: A61M 5/142, A61K 9/00, A61M 5/145, A61M 5/172, A61M 31/00

(54) **IMPLANTABLE DEVICE ACTUATED USING MOTOR AND OSMOTIC MOTOR**

(30) Priority: 23.07.2024 US 202418781451; 23.07.2024 US 202418781485; 02.09.2024 US 202463689805 P; 16.10.2024 US 202463707896 P; 29.10.2024 US 202463713149 P; 31.12.2024 US 202419006285; 31.12.2024 US 202419006301; 31.12.2024 US 202419006316; 31.12.2024 US 202419006338; 31.12.2024 WO PCT/US2024/062372; 31.12.2024 WO PCT/US2024/062373; 31.12.2024 WO PCT/US2024/062374; 31.12.2024 WO PCT/US2024/062371; 01.01.2025 US 202563741032 P; 01.01.2025 US 202563741030 P; 01.01.2025 US 202563741033 P; 01.01.2025 US 202563741031 P; 02.01.2025 US 202563741156 P; 02.01.2025 US 202519007650; 02.01.2025 WO PCT/US2025/010030; 15.01.2025 US 202563745419 P; 15.01.2025 US 202563745416 P; 21.01.2025 US 202563747416 P; 21.01.2025 US 202563747421 P; 22.01.2025 US 202563748085 P; 22.01.2025 US 202563748088 P; 22.01.2025 US 202563748096 P; 22.01.2025 US 202563748104 P; 29.01.2025 US 202563751248 P; 29.01.2025 US 202563750914 P; 29.01.2025 US 202563750879 P; 29.01.2025 US 202563750912 P; 29.01.2025 US 202563750886 P; 29.01.2025 US 202563750895 P; 29.01.2025 US 202563750893 P; 29.01.2025 US 202563750917 P; 29.01.2025 US 202563750921 P; 29.01.2025 US 202563750892 P; 30.01.2025 US 202563751298 P; 06.04.2025 US 202563784181 P; 24.04.2025 US 202563793731 P; 24.04.2025 US 202563793737 P; 30.04.2025 US 202563797341 P
(71) Applicant: Manta MedTech LLC, Newark, Delaware 19702 (US)
(72) Inventor: DAVE, Raju S., Gaithersburg, 20878 (US); SU, Xing, Santa Clara, 95054 (US); VERMA, Himanshu, McLean, 22101 (US)
(74) Representative: Schmidt, Christian

(57) **Abstract**

An active implantable medical device (AIMD) is described. The AIMD comprises: a drug chamber that comprises a drug; a motor chamber that comprises a housing, a motor, and a driving component; a piston that is affixed to a first end of the driving component; and an electronic module that actuates the motor to operate the driving component and move the piston longitudinally to discharge the drug outside the AIMD through one or more orifices.

## Description

### CROSS REFERENCE TO RELATED APPLICATIONS

This application claims priority from some or all of the following applications shown in the Tables below. The disclosure content of all of the applications listed in the Tables below (including applications from which priority is claimed and application from which no priority is claimed for the present application) is related to the present application and is incorporated by reference herein in its entirety.

| **US Patent Application Nos.** | **Filing Date** | **Title of the Invention** |
|---|---|---|
| 63/617,054 | 02-JAN-2024 | PERSONALIZED DRUG DOSING |
| 63/617,057 | 02-JAN-2024 | INNOVATIVE IMPLANTABLE DEVICE USING DOSING CONTROL TO PREVENT MEDICATION ERRORS |
| 63/617,754 | 04-JAN-2024 | IMPLANTABLE DEVICE FOR PRECISION DOSING |
| 63/566,519 | 18-MARCH-2024 | IMPLANTABLE DRUG DELIVERY DEVICE |
| 63/574,330 | 04-APRIL-2024 | INNOVATIVE IMPLANTABLE DEVICE USING DOSING CONTROL TO PREVENT MEDICATION ERRORS |
| 63/661,654 | 19-JUNE-2024 | DEVICE BASED TREATMENTS FOR SUBSTANCE USE DISORDERS |
| 18/781,451 | 23-JULY-2024 | ARTIFICIAL INTELLIGENCE BASED IMPLANTABLE DRUG DELIVERY SYSTEM |
| 18/781,485 | 23-JULY-2024 | DEVICE AND METHOD TO ASSESS RECTAL EFFLUENT |
| 63/689,805 | 02-SEP-2024 | IMPLANTABLE DEVICE FOR DRUG DELIVERY |
| 63/707,896 | 16-OCT-2024 | DEVICE BASED TREATMENTS FOR SUBSTANCE USE DISORDERS |
| 63/713,149 | 29-OCT-2024 | AUTOMATED OVERDOSE RESPONSE SYSTEM FOR ON-DEMAND NALOXONE DELIVERY |
| 19/006,285 | 31-DEC-2024 | ACTIVE IMPLANTABLE MEDICAL DEVICE (AIMD) SYSTEM FOR DRUG DELIVERY WITH FIXED DOSE SIZE |
| 19/006,301 | 31-DEC-2024 | IMPLANTABLE DEVICE FOR ADJUSTABLE DOSE USING PRESSURE SENSOR FOR PISTON DISPLACEMENT MONITORING |
| 19/006,316 | 31-DEC-2024 | IMPLANTABLE DEVICE HAVING PISTON DISPLACEMENT MONITORING |
| 19/006,338 | 31-DEC-2024 | IMPLANTABLE DEVICE ACTUATED USING LEAD SCREW AND MOTOR |
| 63/741,032 | 01-JAN-2025 | IMPLANTABLE DEVICE HAVING PISTON DISPLACEMENT MONITORING |
| 63/741,030 | 01-JAN-2025 | ACTIVE IMPLANTABLE MEDICAL DEVICE (AIMD) SYSTEM FOR DRUG DELIVERY WITH FIXED DOSE SIZE |
| 63/741,033 | 01-JAN-2025 | IMPLANTABLE DEVICE ACTUATED USING LEAD SCREW AND MOTOR |
| 63/741,031 | 01-JAN-2025 | IMPLANTABLE DEVICE FOR ADJUSTABLE DOSE USING PRESSURE SENSOR FOR PISTON DISPLACEMENT MONITORING |
| 63/741,156 | 02-JAN-2025 | AUTOPILOT DRUG-DELIVERY SYSTEM |
| 19/007,650 | 02-JAN-2025 | AUTOPILOT DRUG-DELIVERY SYSTEM |
| 63/745,416 | 15-JAN-2025 | DEVICE AND METHOD TO ASSESS RECTAL EFFLUENT |
| 63/745,419 | 15-JAN-2025 | ARTIFICIAL INTELLIGENCE BASED IMPLANTABLE DRUG DELIVERY SYSTEM |
| 63/747,416 | 21-JAN-2025 | MEDICAL SYSTEM AGAINST A DRUG OVERDOSE |
| 63/747,421 | 21-JAN-2025 | IMPLANTABLE DRUG DELIVERY DEVICE |
| 63/748,088 | 22-JAN-2025 | IMPLANTABLE DRUG DELIVERY SYSTEMS |
| 63/748,085 | 22-JAN-2025 | SYNERGISTIC INTEGRATION OF IMPLANTABLE DRUG DELIVERY PLATFORM WITH WEARABLE MICRONEEDLE SENSORS |
| 63/748,096 | 22-JAN-2025 | IMPLANTABLE DEVICE HAVING PISTON DISPLACEMENT MONITORING |
| 63/748,104 | 22-JAN-2025 | IMPLANTABLE DEVICE ACTUATED USING LEAD SCREW AND MOTOR |
| 63/751,248 | 29-JAN-2025 | DEVICE AND METHOD TO ASSESS RECTAL EFFLUENT |
| 63/750,914 | 29-JAN-2025 | AUTOPILOT DRUG-DELIVERY SYSTEM |
| 63/750,879 | 29-JAN-2025 | IMPLANTABLE DRUG DELIVERY PLATFORM WITH WEARABLE MICRONEEDLE SENSORS |
| 63/750,912 | 29-JAN-2025 | IMPLANTABLE DEVICE ACTUATED USING LEAD SCREW AND MOTOR |
| 63/750,886 | 29-JAN-2025 | IMPLANTABLE DRUG DELIVERY DEVICE |
| 63/750,895 | 29-JAN-2025 | IMPLANTABLE DEVICE HAVING PISTON DISPLACEMENT MONITORING |
| 63/750,893 | 29-JAN-2025 | MEDICAL SYSTEM AGAINST A DRUG OVERDOSE |
| 63/750,917 | 29-JAN-2025 | IMPLANTABLE DEVICE FOR ADJUSTABLE DOSE USING PRESSURE SENSOR FOR PISTON DISPLACEMENT MONITORING |
| 63/750,921 | 29-JAN-2025 | ACTIVE IMPLANTABLE MEDICAL DEVICE (AIMD) SYSTEM FOR DRUG DELIVERY WITH FIXED DOSE SIZE |
| 63/750,892 | 29-JAN-2025 | IMPLANTABLE DRUG DELIVERY SYSTEMS |
| 63/751,298 | 30-JAN-2025 | DRUG FORMULATION CONTAINING CORE-SHELL PARTICLES |
| 63/784,181 | 06-APRIL-2025 | ACTIVE IMPLANTABLE MEDICAL DEVICE FOR EXENATIDE DELIVERY IN TREATMENT OF TYPE 2 DIABETES |
| 63/793,737 | 24-APRIL-2025 | ARTIFICIAL INTELLIGENCE BASED IMPLANTABLE DRUG DELIVERY SYSTEM |
| 63/793,731 | 24-APRIL-2025 | DEVICE AND METHOD TO ASSESS RECTAL EFFLUENT |
| 63/797,341 | 30-APRIL-2025 | ADAPTIVE RECOVERY SYSTEM FOR PERSONALIZED SUPPORT & PROGRESS TRACKING |

| **PCT Patent Application Nos.** | **Filing Date** | **Title of the Invention** |
|---|---|---|
| PCT/US2024/062372 | 31-DEC-2024 | IMPLANTABLE DEVICE FOR ADJUSTABLE DOSE USING PRESSURE SENSOR FOR PISTON DISPLACEMENT MONITORING |
| PCT/US2024/062373 | 31-DEC-2024 | IMPLANTABLE DEVICE HAVING PISTON DISPLACEMENT MONITORING |
| PCT/US2024/062374 | 31-DEC-2024 | IMPLANTABLE DEVICE HAVING PISTON DISPLACEMENT MONITORING |
| PCT/US2024/062371 | 31-DEC-2024 | ACTIVE IMPLANTABLE MEDICAL DEVICE (AIMD) SYSTEM FOR DRUG DELIVERY WITH FIXED DOSE SIZE |
| PCT/US2025/010030 | 02-JAN-2025 | AUTOPILOT DRUG-DELIVERY SYSTEM |

### TECHNICAL FIELD

This disclosure relates to active implantation medical device (AIMD), and more specifically relates to an active implantable medical device that actuates a piston longitudinally using a motor.

### BACKGROUND ART

Poor medication adherence is a significant issue, leading to numerous hospital admissions and high healthcare costs. Factors contributing to nonadherence include poor insight, substance abuse, negative attitudes, side effects, and cognitive impairments. This problem is exacerbated among older adults on multiple medications, resulting in worsened health outcomes and increased mortality. Additionally, drug overdoses, particularly opioid-related, remain a severe public health crisis in the U.S., causing significant loss of life and economic burden.

Considering knowledge of a person skilled in the art, there is a long-felt need to address the shortcomings in the prior art and provide a system that is capable of implementing comprehensive strategies addressing issues related to clinical efficacy, toxicity, drug properties, and personalized drug dosing. It would be advantageous to have a system, method and device that considers at least some of the issues discussed above, as well as possibly other issues.

### SUMMARY OF INVENTION

The following paragraphs present a summary to provide a basic understanding of one or more embodiments described herein. This summary is not intended to identify key or critical elements or delineate any scope of the different embodiments and/or any scope of the claims. The sole purpose of the summary is to present some concepts in a simplified form as a prelude to the more detailed description presented herein.

Embodiments relate to an active implantable medical device (AIMD). The AIMD comprises a drug chamber, a motor chamber, a piston, and an electronic module. The drug chamber comprises a drug. The motor chamber comprises a housing, a motor, and a driving component. The motor may be a stepper motor. The driving component is coupled to the motor such that when the motor is operated the driving component also rotates. The driving component may be a helical threaded screw which converts the rotational motion to a linear motion. The piston is affixed to a first end of the driving component. The first end is an end which is towards one or more orifices through which the drug is discharged from the AIMD to outside the AIMD (i.e., a subject). As the piston is affixed to the first end of the driving component, the piston moves longitudinally towards the one or more orifices, which discharges the drug from the drug chamber to the subject (i.e., outside the AIMD). The subject may be a living being (e.g., a human, animal, a mammal, etc.).

The active implantable medical device (AIMD) may comprise a sensor module and an electronic module. The sensor module is configured to measure displacement of a piston. The electronic module communicates a signal to the stepper motor to control the stepper motor and regulate the one-way flow of the drug based on the displacement of the piston precisely and accurately. Additionally, the electronic module comprises a power supply module for supplying energy to the electronics module, the sensor module, and the motor. The device is designed with a tubular structure for subcutaneous implantation. The device releases the drug, while the sensor module monitors relevant parameters, for example, displacement. A communication web connects all modules, ensuring the synchronized movement of the piston towards the drug chamber with each release, with motor the actuating this action and the sensor detecting the piston's movement.

In an aspect, an active implantable medical device (AIMD) is described. The AIMD comprises: a drug chamber that comprises a drug; a motor chamber that comprises a housing, a motor, and a driving component; a piston that is affixed to a first end of the driving component; and an electronic module that actuates the motor to operate the driving component and move the piston longitudinally to discharge the drug outside the AIMD through one or more orifices.

In some embodiments, the driving component comprises a helical threaded screw. A first length of the driving component is equal to the sum of a second length of the motor, a third length between an initial position of the piston and a motor position, and a fourth length between the initial position of the piston and a displacement position of the piston.

The AIMD may comprise a check valve coupled to the one or more orifices. The check valve allows one-way flow of the drug from the drug chamber to outside the AIMD through the one or more orifices. The check valve blocks entry of a fluid to the drug chamber from outside the AIMD. The check valve remains closed until the incoming pressure reaches a threshold pressure.

In some embodiments, the piston and the driving component moves together longitudinally to discharge the drug upon actuating the motor. An outer circumference of the piston is in surface compliance with the AIMD to prevent leakage or contamination.

The AIMD comprises a sensor module. In an embodiment, the sensor module monitors one or more physiological parameters of a subject. The sensor module communicates a signal to the electronic module when the one or more physiological parameters of the subject is abnormal. The sensor module may communicate a signal to the electronic module at a prescribed time. The electronic module may actuate the motor in response to a signal received from the sensor module.

In some embodiments, an outer circumference of the piston comprises a sealing component. The sealing component prevents leakage of the drug from the drug chamber to the motor chamber. The sealing component prevents contamination of the drug in the drug chamber with a fluid or gas in the motor chamber.

In some embodiments, the motor chamber is configured to pressurize a gas within the motor chamber of the AIMD. The pressurization of the gas restricts creation of vacuum within the AIMD when the piston moves longitudinally towards the one or more orifices. The gas may be an inert gas.

In some embodiments, the housing of the motor chamber comprises a permeability module. The permeability module comprises a semipermeable membrane and an osmotic agent chamber. The permeability module allows ingress flow of a fluid to an osmotic agent chamber and generates osmotic pressure. The osmotic pressure drives the piston to move longitudinally towards the one or more orifices in addition to the actuation of the motor. The osmotic pressure is directly proportionate to the movement of the piston longitudinally. The ingress flow of the fluid controls the discharge of the drug.

In some embodiments, the housing of the motor chamber comprises one or more provisions and a filter. The one or more provisions allows ingress flow of a fluid from outside the AIMD to the motor chamber through the filter. The ingress flow of the fluid from outside the AIMD to the motor chamber prevents creation of vacuum within the AIMD.

In an embodiment, the electronic module comprises a microprocessor.

In another aspect, an active implantable medical device (AIMD) is described. The AIMD comprises: a drug chamber that comprises a drug; a motor chamber that comprises a housing, a motor, and a driving component; a stationary guiding component comprising a provision through which the driving component protrudes longitudinally; and an electronic module that actuates the motor to operate the driving component and protrude longitudinally through the stationary guiding component to discharge the drug outside the AIMD through one or more orifices. The electronic module may comprise a microprocessor. The AIMD may comprise a sealing component between the stationary guiding component and the driving component.

The driving component may comprise a flattened surface at a first end to push the drug through the one or more orifices. An outer circumference of the flattened surface is in surface compliance with the AIMD. The driving component is operable to function as a piston to discharge the drug outside the AIMD through one or more orifices. The driving component comprises a helical threaded screw. The length of the driving component is equal to the sum of a length of the motor, a length between an initial position of the first end of the driving component and a motor position, and a length between the initial position of the first end of the driving component and a displacement position of the first end of the driving component. The stationary guiding component comprises one or more bumps configured to prevent rotation of the stationary guiding component. An outer circumference of the stationary guiding component comprises a sealing component. The sealing component prevents leakage of the drug from the drug chamber to the motor chamber. The sealing component prevents contamination of the drug in the drug chamber with a fluid or gas in the motor chamber.

The AIMD may comprise a check valve coupled to the one or more orifices. The check valve allows one-way flow of the drug from the drug chamber to outside the AIMD through the one or more orifices. The check valve blocks entry of a fluid to the drug chamber from outside the AIMD.

The AIMD comprises a sensor module. The sensor module monitors one or more physiological parameters of a subject. The sensor module communicates a signal to the electronic module when the one or more physiological parameters of the subject is abnormal. The sensor module communicates a signal to the electronic module at a prescribed time. The electronic module actuates the motor in response to a signal received from the sensor module.

In some embodiments, the motor chamber is configured to pressurize a gas within the motor chamber of the AIMD. The pressurization of the gas restricts creation of vacuum within the AIMD when the driving component moves longitudinally towards the one or more orifices. The gas may be an inert gas.

In some embodiments, the housing of the motor chamber comprises a permeability module. The permeability module comprises a semipermeable membrane and an osmotic agent chamber. The permeability module allows ingress flow of a fluid to an osmotic agent chamber and generates osmotic pressure. The osmotic pressure pushes the driving component to move longitudinally towards the one or more orifices in addition to the actuation of the motor. The osmotic pressure is directly proportionate to the movement of the driving component longitudinally. The ingress flow of the fluid controls the discharge of the drug.

In some embodiments, the housing of the motor chamber comprises one or more provisions and a filter. The one or more provisions allows ingress flow of a fluid from outside the AIMD to the motor chamber through the filter. The ingress flow of the fluid from outside the AIMD to the motor chamber prevents creation of vacuum within the AIMD.

In another aspect, a method is described. The method comprises: monitoring one or more physiological parameters of a subject using a sensor module; communicating a signal to an electronic module when the one or more physiological parameters is abnormal; and actuating a motor using the electronic module based on the signal to operate a driving component and move a piston longitudinally to discharge a drug to a subject outside an active implantable medical device (AIMD) through one or more orifices. The sensor module may be placed within the subject. The sensor module is communicatively coupled to the AIMD.

The methods and systems disclosed herein may be implemented by any means necessary for achieving various aspects to perform any of the operations disclosed herein. Other features will be apparent from the accompanying drawings and from the detailed description that follows.

### BRIEF DESCRIPTION OF DRAWINGS

These and other aspects of the present disclosure will now be described in more detail, with reference to the appended drawings showing exemplary embodiments of the present disclosure, in which:
**FIG. 1** illustrates an active implantable medical device (AIMD) comprising a lead screw and a motor for actuating a piston in longitudinal direction, according to one or more embodiments.
**FIG. 2A** illustrates an AIMD comprising a check valve, according to one or more embodiments.
**FIG. 2B** depicts a schematic diagram of the check valve shown in FIG. 2A.
**FIG. 3A** illustrates an AIMD comprising a check valve, according to one or more embodiments.
**FIG. 3B** depicts a schematic diagram of the check valve shown in FIG. 3A.
**FIG. 4** illustrates a lead screw and motor arrangement, according to one or more embodiments.
**FIG. 5A** illustrates an AIMD comprising a moving grooved cylinder on lead screw, according to one or more embodiments.
**FIG. 5B** illustrates an AIMD comprising a moving piston on lead screw, according to one or more embodiments.
**FIG. 5C** illustrates an AIMD comprising a moving cylinder using gears, according to one or more embodiments.
**FIG. 5D** illustrates an AIMD comprising a submersible pump, according to one or more embodiments.
**FIG. 5E** illustrates an AIMD comprising a submersible pump and a compressible drug chamber, according to one or more embodiments.
**FIG. 6A** illustrates an AIMD comprising a semipermeable membrane on a motor chamber, according to one or more embodiments.
**FIG. 6B** illustrates a schematic of a flat membrane as a permeability module, according to one or more embodiments.
**FIG. 6C** illustrates a schematic of hollow fiber membrane as a permeability module, according to one or more embodiments.
**FIG. 7** illustrates an AIMD comprising one or more provisions on a motor chamber to allow flow of interstitial fluid and prevent creation of vacuum, according to one or more embodiments.
**FIG. 8** illustrates an AIMD that pressurizes gas on a motor chamber to prevent creation of vacuum, according to one or more embodiments.
**FIG. 9** illustrates a method of actuating a motor and discharging a drug, according to one or more embodiments.
**FIG. 10** illustrates a non-transitory computer readable storage medium, according to one or more embodiments.

Other features of the present embodiments will be apparent from the accompanying drawings and from the detailed description that follows.

### DESCRIPTION OF EMBODIMENTS

### Definitions and General Techniques

For simplicity and clarity of illustration, the drawing figures illustrate the general manner of construction, and descriptions and details of well-known features and techniques may be omitted to avoid unnecessarily obscuring the present disclosure. Additionally, elements in the drawing figures are not necessarily drawn to scale. For example, the dimensions of some of the elements in the figures may be exaggerated relative to other elements to help improve understanding of embodiments of the present disclosure. The same reference numerals in different figures denote the same elements.

The terms "first," "second," "third," "fourth," and the like in the description and in the claims, if any, are used for distinguishing between similar elements and not necessarily for describing a particular sequential or chronological order. It is to be understood that the terms so used are interchangeable under appropriate circumstances such that the embodiments described herein are, for example, capable of operation in sequences other than those illustrated or otherwise described herein. Furthermore, the terms "include," and "have," and any variations thereof, are intended to cover a non-exclusive inclusion, such that a process, method, system, article, device, or apparatus that comprises a list of elements is not necessarily limited to those elements, but may include other elements not expressly listed or inherent to such process, method, system, article, device, or apparatus.

The terms "left," "right," "front," "back," "top," "bottom," "over," "under," and the like in the description and in the claims, if any, are used for descriptive purposes and not necessarily for describing permanent relative positions. It is to be understood that the terms so used are interchangeable under appropriate circumstances such that the embodiments of the apparatus, methods, and/or articles of manufacture described herein are, for example, capable of operation in other orientations than those illustrated or otherwise described herein.

No element, act, or instruction used herein should be construed as critical or essential unless explicitly described as such. Also, as used herein, the articles "a" and "an" are intended to include items and may be used interchangeably with "one or more." Furthermore, as used herein, the term "set" is intended to include items (e.g., related items, unrelated items, a combination of related items, and unrelated items, etc.), and may be used interchangeably with "one or more." Where only one item is intended, the term "one" or similar language is used. Also, as used herein, the terms "has," "have," "having," or the like are intended to be open-ended terms. Further, the phrase "based on" is intended to mean "based, at least in part, on" unless explicitly stated otherwise.

The terms "couple," "coupled," "couples," "coupling," and the like should be broadly understood and refer to connecting two or more elements mechanically and/or otherwise. Two or more electrical elements may be electrically coupled together, but not be mechanically or otherwise coupled together. Coupling may be for any length of time, e.g., permanent, or semipermanent or only for an instant. "Electrical coupling" and the like should be broadly understood and include electrical coupling of all types. The absence of the word "removably," "removable," and the like near the word "coupled," and the like does not mean that the coupling, etc., in question is or is not removable.

As defined herein, two or more elements are "integral" if they are comprised of the same piece of material. As defined herein, two or more elements are "non-integral" if each is comprised of a different piece of material.

As defined herein, "real-time" can, in some embodiments, be defined with respect to operations carried out as soon as practically possible upon occurrence of a triggering event. A triggering event can include receipt of data necessary to execute a task or to otherwise process information. Because of delays inherent in transmission and/or in computing speeds, the term "real-time" encompasses operations that occur in "near" real-time or somewhat delayed from a triggering event. In a number of embodiments, "real-time" can mean real-time less a time delay for processing (e.g., determining) and/or transmitting data. The particular time delay can vary depending on the type and/or amount of the data, the processing speeds of the hardware, the transmission capability of the communication hardware, the transmission distance, etc. However, in many embodiments, the time delay can be less than approximately one second, two seconds, five seconds, or ten seconds.

The present disclosure may be embodied in other specific forms without departing from its spirit or characteristics. The embodiments described are to be considered in all respects only as illustrative and not restrictive. The scope of the disclosure is, therefore, indicated by the appended claims rather than by the foregoing description. All changes which come within the meaning and range of equivalency of the claims are to be embraced within their scope.

As defined herein, "approximately" can, in some embodiments, mean within plus or minus ten percent of the stated value. In other embodiments, "approximately" can mean within plus or minus five percent of the stated value. In further embodiments, "approximately" can mean within plus or minus three percent of the stated value. In yet other embodiments, "approximately" can mean within plus or minus one percent of the stated value.

Unless otherwise defined herein, scientific, and technical terms used in connection with the present disclosure shall have the meanings that are commonly understood by those of ordinary skill in the art. Further, unless otherwise required by context, singular terms shall include pluralities and plural terms shall include the singular. Generally, nomenclatures used in connection with, and techniques of, health monitoring described herein are those well-known and commonly used in the art.

The methods and techniques of the present disclosure are generally performed according to conventional methods well-known in the art and as described in various general and more specific references that are cited and discussed throughout the present specification unless otherwise indicated. The nomenclatures used in connection with, and the procedures and techniques of, embodiments herein, and other related fields described herein are those well-known and commonly used in the art.

The following terms and phrases, unless otherwise indicated, shall be understood to have the following meanings.

As used herein, the term "drug chamber" refers to a compartment, container, or enclosed space within the AIMD designed for the controlled storage, dispensing, or delivery of drugs or pharmaceutical substances.

As used herein, the term "motor chamber" refers to a housing or enclosure that contains a motor and related components. The motor chamber serves to protect the motor from environmental factors and ensure its proper functioning.

As used herein, the term "driving component" refers to a component that links the piston to a motor shaft. The driving component may comprise helical threaded region which converts the rotational motion to longitudinal motion and moves the piston longitudinally and discharges the drug outside the AIMD.

As used herein, the term "lead screw" also known as a power screw or translation screw, is a mechanical device used to convert rotational motion into linear motion. The screw has a helical thread that engages with a corresponding threaded nut, moving it along the axis of the screw. Lead screws come in various thread forms, for example trapezoidal, ACME, and square threads. Lead screws are used in applications requiring precise linear positioning.

As used herein, the term "medication" is defined as a substance or combination of substances or drug used to diagnose, treat, prevent, or alleviate symptoms of a disease or medical condition.

As used herein, the term "interstitial fluid" refers to a medium through which devices interact with the body. Interstitial fluid provides a direct interface between the device and the body. The AIMD relies on interstitial fluid for the diffusion of drugs into surrounding tissues. Interstitial fluid can act as a trigger or medium for dynamic responses in implantable devices. The interstitial fluid acts as a balancing agent, maintaining stable pressure within the device and enabling the piston to move forward without hindrance, ensuring precise and continuous medication delivery.

As used herein, the term "housing" refers to a protective enclosure or framework that surrounds and supports a component, device, or system. Its primary purpose is to shield the internal parts from external elements, provide structural integrity, and sometimes aid in heat dissipation, noise reduction, or aesthetic appeal.

As used herein, the term "piston" refers to a precision-engineered component to control the movement of fluids. Pistons are used to regulate the precise delivery of medication by moving in response to pressure changes or external controls.

As used herein, the term "electronic module" refers to a compact and integrated unit containing the electronic components and circuits required for the device to perform its functions. The electronic module is configured to perform control and processing, data storage, communication, and power management. The electronic module is designed considering the Hermetic Sealing to protect the electronics from body fluids and to ensure longevity.

As used herein, the term "helical thread" refers to a spiral-shaped groove or ridge that wraps around a surface of a component. It is a key feature of screws, bolts, and other threaded fasteners, as well as components like leadscrews or threaded pipes. Helical threads are adapted to convert rotational motion into linear motion, and to form a tight seal between components.

As used herein, the term "check valve" refers to a mechanical device designed to allow the flow of fluids (liquids or gases) in one direction only, preventing reverse flow. It is a type of non-return valve that operates automatically, relying on the pressure and flow of the fluid to open or close the valve. The check valve may be one of swing check valve, ball check valve, lift check valve, diaphragm check valve, and spring-loaded valve. The check valve involves minimal maintenance due to uncomplicated design. The check valve remains closed until the incoming pressure reaches a threshold pressure.

As used herein, the term "orifice" refers to a small, precisely sized opening or hole in a plate, pipe, or other structure, designed to control or regulate the flow of fluids (liquids or gases). Orifices are widely used in various applications to manage flow rates, create pressure drops, or measure flow quantities.

As used herein, the term "longitudinal" refers to something that is oriented or occurring along the length or axis of an object or system.

As used herein, the term "sensor module" refers to a compact, integrated unit that contains one or more sensors along with the necessary circuitry to measure, process, and sometimes transmit data. These modules are designed to detect specific physical properties (such as piston displacement, physiological parameters) and convert them into readable signals, which can be interpreted by a system or device.

As used herein, the term "actuate" refers to causing a mechanism or device to operate or set it into motion.

As used herein, the term "sealing component" refers to a part used to create a tight seal between two surfaces, preventing the leakage of fluids (liquids or gases), dust, or contaminants. Sealing components are essential in maintaining the integrity, safety, and performance of medical devices.

As used herein, the term "pressurization" refers to a process of increasing or maintaining the pressure of a gas or fluid within a confined space, system, or environment.

As used herein, the term "inert gas" refers to a gas that is chemically unreactive or highly stable under normal conditions. These gases do not easily form chemical compounds because their atoms have complete electron shells, making them less likely to participate in chemical reactions. Inert gases are used in medical devices where reactions need to be prevented.

As used herein, the term "osmotic pressure" refers to a pressure exerted by a solvent as it passes through a semipermeable membrane to equalize the concentration of solute on both sides of the membrane. This phenomenon occurs during osmosis, where solvent molecules move from a region of lower solute concentration to a region of higher solute concentration, in order to balance the concentrations on either side of the membrane.

As used herein, the term "semipermeable membrane" refers to a membrane that allows some molecules or ions to pass through while blocking others. The key characteristic of a semipermeable membrane is that it selectively permits the movement of certain substances, usually based on size, charge, or other properties, while preventing the passage of larger or differently charged molecules.

As used herein, the term "provision" refers to mechanism, design, or tool providing or supplying something. The provisions may be holes.

As used herein, the term "microprocessor" refers to a small, integrated electronic component that serves as the central processing unit (CPU) of the device, responsible for managing and controlling its functions. The microprocessor plays a critical role in ensuring that the device operates according to specific medical protocols. It processes data from sensors (e.g., piston displacement, blood pressure, glucose levels, or heart rate), makes decisions based on programmed algorithms, and can activate mechanisms like drug delivery systems, stimulation, or external communication.

As used herein, the term "surface compliance" refers to the ability of a material, structure, or component to deform or adapt to the surface it comes into contact with. In other words, it is a measure of how much a surface or object can bend or yield under external pressure or force, allowing it to conform to irregularities or uneven surfaces.

As used herein, the term "stationary guiding component" refers to a fixed part or structure designed to direct or control the movement of the driving component along a specific path or axis. The stationary guiding component helps to maintain precise movement, alignment, or positioning of the driving component without moving itself.

The term "power source" refers to a component that could provide necessary energy to operate a device. Typically, this is a battery housed within the system's case.

The term "microprocessor" (MCU for microcontroller unit) is a small computer on a single metal-oxide-semiconductor (MOS) integrated circuit (IC) chip. A microprocessor contains one or more CPUs (processor cores) along with memory and programmable input/output peripherals. Program memory in the form of ferroelectric Random Access Memory (RAM), Not OR (NOR) flash or One-Time Programmable Read-Only Memory (OTP ROM) is also often included on chip, as well as a small amount of RAM.

The microprocessors may be designed for embedded applications. The microprocessors may comprise various discrete chips.

The term "microprocessor" may be used interchangeably with terms such as "controller," "processor" or "microcontroller" and like.

The term "physiological parameters" include, but are not limited to, subject body temperature, subject heart rate, subject heart rate variability, subject blood gas levels, subject metabolic rate, subject respiration rate, subject blood analyte levels, subject blood pressure, subject pulse pressure, etc.

The term "Bluetooth Low Energy" (BLE) is a wireless personal area network (WPAN) technology designed and marketed by the Bluetooth Special Interest Group (Bluetooth SIG) aimed at novel applications in the healthcare, fitness, beacons, security, and home entertainment industries. It is independent of Bluetooth BR/EDR and has no compatibility, but Basic Rate/ Enhanced Data Rate (BR/EDR) and Low Energy (LE) can coexist. The original specification was developed by Nokia in 2006 under the name Wibree which was integrated into Bluetooth 4.0 in December 2009 as Bluetooth Low Energy. All wireless personal area networks (WPANs) including Recommended Dietary Allowance (rDA), Wireless Universal Serial Bus (USB), Bluetooth or ZigBee come under the scope of the present invention.

The term "sensor" is a device, module, machine, or subsystem whose purpose is to detect events or changes in its environment and send the information to other electronics, frequently a computer processor. A sensor is always used with other electronics.

The term "semipermeable membrane" is a type of biological, synthetic, or polymeric membrane that allows certain molecules or ions to pass through it by diffusion-or occasionally by more specialized processes of facilitated diffusion, passive transport, or active transport. The rate of passage depends on the pressure, concentration, and temperature of the molecules or solutes on either side, as well as the permeability of the membrane to each solute. Depending on the membrane and the solute, permeability may depend on solute size, solubility, properties, or chemistry.

Embodiments of the invention are applicable to humans and more generally to mammals (host). Present disclosure provides a device for controlled delivery of drugs. "Drug" in context of the present disclosure may include any therapeutic active agent and/or a biologically active agent (i.e., an active ingredient in a pharmaceutical composition that is biologically active, such as a vaccine). "Drug" in context of the present disclosure is not limited by molecular weight of such agents. Terms "drug," "active agent," "therapeutic agent," "beneficial agent," or "pharmaceutical fluid" are used interchangeably. Drug as used herein refers to a single drug or multiple types of drugs. In some embodiments, the drug is one of an injectable drug such as without limitation Adalimumab, dulaglutide, enosumab, ustekinumab, pneumococcal 13-valent vaccine, romiplostim, paliperidone palmitate, erenumab, benralizumab, ixekizumab, ofatumumab, pegfilgrastim, guselkumab, golimumab, asfotase alfa, and blinatumomab. In some embodiments, the drug is a repurposed drug such as without limitation apixaban, lenalidomide, semaglutide, rivaroxaban, dapagliflozin, pomalidomide, fingolimod, ozanimod, tofacitinib, ambrisentan, axitinib, lenvatinib, and cariprazine.

Embodiments relate to an active implantable medical device (AIMD). The AIMD comprises a drug chamber, a motor chamber, a piston, and an electronic module. The drug chamber comprises a drug. The motor chamber comprises a housing, a motor, and a driving component. The motor may be a stepper motor. The driving component is coupled to the motor via a motor shaft such that when the motor is operated the driving component also rotates. The driving component may be a helical threaded screw which converts the rotational motion to a linear motion. The piston is affixed to a first end of the driving component. The first end is an end which is towards one or more orifices through which the drug is discharged from the AIMD to outside the AIMD (i.e., a subject). As the piston is affixed to the first end of the driving component, the piston moves longitudinally towards the one or more orifices, which discharges the drug from the drug chamber to the subject (i.e., outside the AIMD). The subject may be a living being (e.g., a human, animal, a mammal, etc.).

The active implantable medical device (AIMD) may comprise a sensor module and an electronic module. The sensor module is configured to measure displacement of a piston. The electronic module communicates a signal to the stepper motor to control the stepper motor and regulate the one-way flow of the drug based on the displacement of the piston. Additionally, the electronic module comprises a power supply module for supplying energy to the electronics module, the sensor module, and the motor. The device is designed with a tubular structure for subcutaneous implantation. The AIMD continuously releases the drug at prescribed times, while the sensor module monitors relevant parameters, for example, displacement. A communication web connects all modules, ensuring the synchronized movement of the piston towards the drug chamber with each release, with the motor actuating this action and the sensor detecting the piston's movement.

In an embodiment, the dose-to-dose variation of a drug via the device is ±25% or less by volume. In an embodiment, the dose-to-dose variation or flow discharge accuracy (used interchangeably throughout the specification) of a drug via the device is ±20% or less by volume. In an embodiment, the device has a dose accuracy of no wider than ±15% of the intended target dose.

In another embodiment, the flow discharge accuracy is ±10%. In another embodiment, the flow discharge accuracy is ±5%. In another embodiment, the flow discharge accuracy is ±3%.

In some embodiments, the AIMD has different modules such as without limitation a drug chamber, a motor chamber, a power module, a valve module, a sensor module, and an electronic module. These modules are interconnected with each other. A person skilled in the art would understand that different ways of interconnecting these modules is possible, such as but not limited to screwing them together, creating notches at the ends of the modules, etc. In some embodiments, each module is threaded with male-female threads such that the first module screws into the second module, the second module screws into the third module, and the third module screws into the fourth module.

In some embodiments, the sensor module and the electronic module could be adhesively bonded to the device tube.

In an embodiment, the AIMD has a tubular structure having an outermost casing. In an embodiment, the casing is made up of a biocompatible material or Food and Drug Administration (FDA) approved material, such as without limitation Titanium. In an embodiment, the casing has housing to connect different modules. As per principle of operation of the implantable, a person skilled in the art would understand that different ways of putting electrical circuits in the casing is possible, such as but not limited to gluing or imprinting. In some embodiments, the housing material provides durability, corrosion resistance, and compatibility with body and with the fluids within the device, such as but not limited to Titanium.

**In** an embodiment, the device tube has an outer diameter between 3 mm to 5 mm, such as 3 mm, 3.5 mm, 4 mm, 4.5 mm, etc. In an embodiment, the casing has a wall thickness about 0.25 mm, 0.3 mm, 0.4 mm, 0.5 mm, 0.6 mm, 0.7 mm, 0.8 mm, 0.1 mm.

**In** another aspect, an active implantable medical device (AIMD) is described. The AIMD comprises a drug chamber, a motor chamber, a stationary guiding component, and an electronic module. The drug chamber comprises a drug. The motor chamber comprises a housing, a motor, and a driving component. The stationary guiding component comprises a provision through which the driving component protrudes longitudinally. The electronic module actuates the motor to operate the driving component and protrude longitudinally through the stationary guiding component to discharge the drug outside the AIMD through one or more orifices. The driving component may comprise a flattened surface at a first end to push the drug through the one or more orifices. The AIMD may comprise a sealing component between the stationary guiding component and the driving component. The driving component is operable to function as a piston to discharge the drug outside the AIMD through one or more orifices. The driving component may comprise a helical threaded screw. The other embodiments and variations described in this disclosure are applicable to this aspect as well.

**Technical Problem:** The convention implantable devices lack efficient and controlled delivery of drugs to a subject, such as a human or animal. The challenge lies in ensuring that the drug is discharged from the drug chamber in a precise and reliable manner. A key issue is the need for a mechanism to move the piston, which discharges the drug, in a controlled way. This requires converting the rotational motion of the motor into the linear motion needed to move the piston longitudinally towards the orifices, thereby releasing the drug.

**Technical Solution to Problem:** The technical solution describes the controlled and precise delivery of a drug from an active implantable medical device (AIMD) to a subject (such as a human or animal). This is achieved through a mechanism that converts rotational motion from a stepper motor into linear motion to move a piston in the drug chamber. The solution involves using a helical threaded screw as the driving component, which rotates when the motor is operated. This rotational motion is converted into linear motion, causing the piston to move longitudinally towards the orifices of the device, thereby discharging the drug from the chamber to the outside (i.e., to the subject). The system ensures accurate and controlled drug delivery by precisely moving the piston and allowing for the regulated release of the drug, addressing the need for effective, reliable drug administration in an implantable medical device.

**Advantageous effects of invention:** The system offers several advantageous effects, including precise and controlled drug delivery, which ensures accurate dosing and reliable release of medication from the drug chamber to the subject. The use of a stepper motor and a helical threaded screw enables precise control over the piston's movement, minimizing the risk of over or under-dosing. This design reduces the need for manual intervention, making the drug administration process more automated and convenient for patients, which in turn enhances patient compliance. The compact and efficient mechanism is ideal for implantable medical devices, ensuring long-term, reliable operation within the body. By minimizing human error and allowing for regulated drug release, the system provides consistent and continuous medication delivery, which is crucial for chronic conditions. Additionally, the device's versatility makes it suitable for a variety of subjects, including humans and animals. Overall, this technology offers a more reliable, convenient, and personalized approach to drug delivery, potentially improving treatment outcomes while reducing healthcare burdens.

### Description of Embodiments:

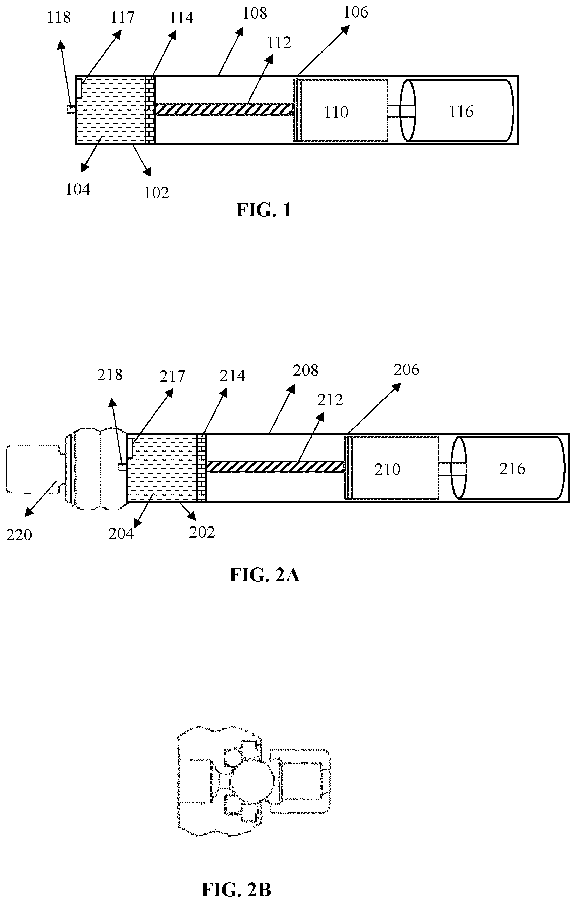

**FIG.** 1 illustrates an active implantable medical device (AIMD) comprising a lead screw and a motor for actuating a piston in longitudinal direction, according to one or more embodiments. The AIMD comprises a drug chamber 102, a motor chamber 106, a piston 114 and an electronic module 116. The drug chamber 102 comprises a drug 104. The motor chamber 106 comprises a housing 108, a motor 110, and a driving component 112. The motor 110 may be a stepper motor or a DC motor. The driving component 112 is coupled to the motor 110 via a motor shaft. The driving components comprise threads that are placed externally onto the body of the driving component 112. The driving component 112 itself may be a helical threaded screw. The motor shaft comprises internal threads. The internal threads of the motor shaft match the threads of the driving component 112 such that when the motor 110 is operated the driving component 112 also rotates. The piston 114 is affixed to the first end of the driving component 112. The motor shaft functions as the guiding component enabling the piston 114 and the driving component 112 to operate together and move longitudinally.

The piston 114 and the driving component 112 move together longitudinally to discharge the drug 104 upon actuating the motor 110. The driving component 112 may rotate and protrude through the motor 110. The driving component 112 may be a helical threaded screw which converts the rotational motion to a linear motion. The driving component 112 comprises a length that is equal to sum of (a) a length of the motor, (b) a length between an initial position of the first end of the driving component and a motor position, and (c) a length between the initial position of the first end of the driving component and a displacement position of the first end of the driving component 112.

The piston 114 is affixed to a first end of the driving component 112. The first end is an end which is towards one or more orifices 118 through which the drug is discharged from the AIMD to outside the AIMD (i.e., a subject). As the piston 114 is affixed to the first end of the driving component 112, the piston 114 and the driving component 112 move together longitudinally towards the one or more orifices 118, which discharges the drug 104 from the drug chamber 102 to the subject (i.e., outside the AIMD). The subject may be a living being (e.g., a human, animal, a mammal, etc.). The outer circumference of the piston 114 comprises a sealing component. The outer circumference of the piston 114 is in surface compliance with the AIMD. The sealing component prevents leakage of the drug from the drug chamber 102 to the motor chamber 106. The sealing component prevents contamination of the drug 104 in the drug chamber 102 with a fluid or gas in the motor chamber 106.

The active implantable medical device may comprise a sensor module 117. The sensor module 117 is configured to measure displacement of the piston 114. The electronic module 116 communicates a signal to the motor 110 to control the motor 110 and regulate the one-way flow of the drug 104 based on the displacement of the piston 114. Additionally, the electronic module 116 comprises a power supply module for supplying energy to the electronics module 116, the sensor module 117, and the motor 110. The device is designed with a tubular structure for subcutaneous implantation. The device continuously releases the drug 104 at prescribed times, while the sensor module 117 monitors relevant parameters, for example, displacement. A communication web connects all modules, ensuring the synchronized movement of the piston towards the drug chamber 102 with each release, with motor 110 actuating this action and the sensor module 117 detecting the movement of the piston 114.

The electronic module 116 may be in a hollow shape or conical shape. The hollow shape or conical shape is adapted to enable the protrusion of the driving component 112 through the electronic module 116 at the back of the motor 110. The conical shape is adapted to achieve a compact sized AIMD. The electronic components may be placed around the path of the driving component 112 at the back of the motor 110.

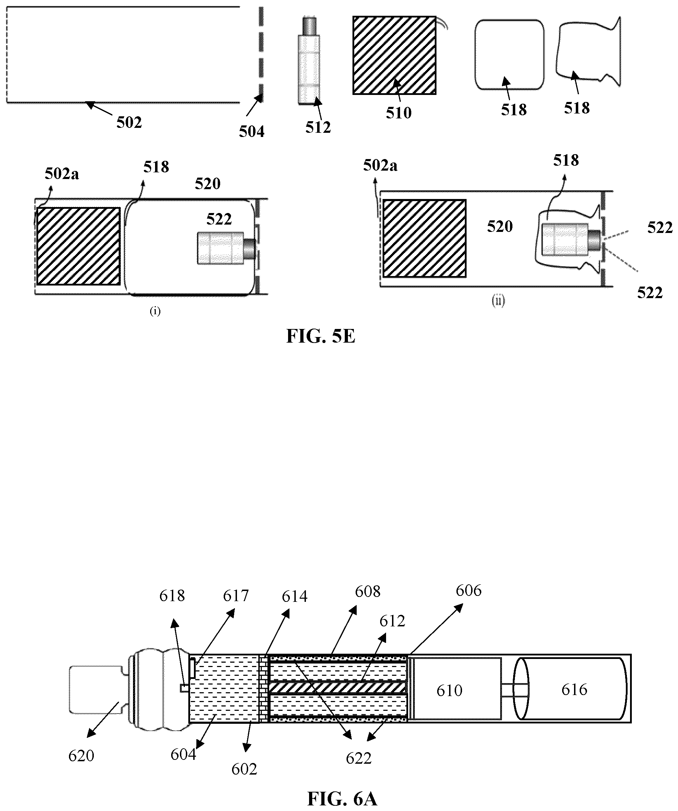

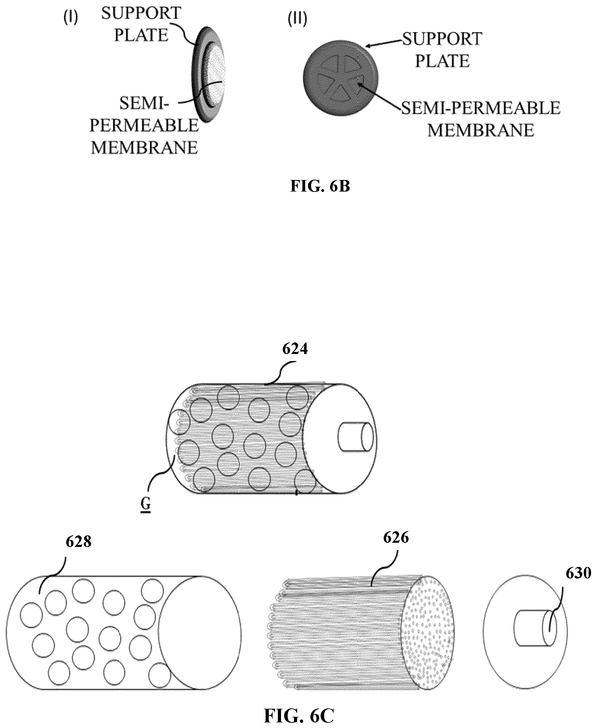

The AIMD may receive interstitial fluid through a channel from one of a fluid storage or from outside the AIMD. The fluid storage may be within the AIMD or outside the AIMD. The interstitial fluid may enter and occupy the space created due to the displacement of the piston 114. The ingress flow or entry of the interstitial fluid restricts the creation of the vacuum in the displacement area of the piston 114. The AIMD may comprise one or more mechanisms (described below in FIG. 6, FIG. 7, and FIG. 8) to allow entry of the interstitial fluid and to restrict creation of vacuum within the AIMD.

In some embodiments, the electronic module 116 comprises chips, electronics housing, and/or electronic components. In some embodiments, the electronic module 116 comprises without limitation one or more of sensors, data storage, communication system, power switch to ensure proper functioning. Sensors continuously or discontinuously monitor various parameters such as drug levels within a patient body and/or within the device, patient vitals, and environmental conditions, etc., to provide real-time data to the microprocessor for processing. The controllers could manage operations such as receiving data from various device components such as sensors and/or battery. The actuator of the controller and the valve module work together to release medication as needed. The communications system, featuring a Bluetooth module, facilitates wireless communication with external devices for data exchange and remote control. Wi-Fi enables internet connectivity for remote monitoring and updates, while inductive and magnetic pulses support wireless charging and communication through the battery charge coil or pump coil. Light sensors detect and communicate using light pulses, and the system also utilizes other known communication methods. In an embodiment, the electronic module may also form a connection with a handheld device that could be used by a user (e.g., patient, caretaker, etc.) to monitor and control the device remotely.

Data logging and storage functionalities ensure that the AIMD logs and retains valuable information such as dosing history, sensor readings, fault conditions, and battery status, which are crucial for monitoring and adjusting treatment. Additional outputs such as electromagnetic fields are used for therapeutic purposes, while light pulses are emitted for communication or therapy.

In an embodiment, the power switch controls the device's power state, allowing it to be turned on or off as needed. In an embodiment, a processor or simple timer manages the device's operations, including recording and adjusting medication dosing based on sensor inputs, monitoring fault conditions, processing sensor data for accurate operation, and overseeing battery status and power usage. In an embodiment, it may also store relevant patient medical history for reference. The system ensures that the AIMD device operates efficiently and effectively, providing necessary treatment while maintaining patient safety.

In some embodiments, the microprocessor (or processor) is configured to analyze the medical characteristics of the patient to determine a symptom associated with a medical condition such as without limitation an opiod overdose, measuring physiological parameters of the user (e.g., patient) of the device. In an embodiment, the microprocessor is configured to analyze medical characteristics of the patient to determine a symptom associated within the patient, wherein the symptom corresponds to a decrease in respiratory rate, a decrease in heart rate, a decrease in blood pressure, deviations from normal body temperature, passing out or an unresponsive loss of consciousness, skin color changes, abnormal breathing, fast, slow or irregular breathing, severe chest pain, seizures, severe headaches, difficulty in breathing, delirium, agitation, and/or anxiety. In some embodiments, the microprocessor is further configured to send an alert or notification wirelessly upon detection of the symptom.

The wireless communication system of the electronic module 116 enables the device to transmit data to external devices, allowing for remote monitoring and adjustments. The microprocessor plays a central role in managing data flow and device control, processing inputs from the sensors and executing commands to the actuator.

In some embodiments, the sensor module 117 monitors one or more physiological parameters of a subject. The sensor module 117 communicates a signal to the electronic module when the one or more physiological parameters of the subject is abnormal. The electronic module 116 actuates the motor in response to a signal received from the sensor module 117. The AIMD further comprises a biosensor coupled to the microcontroller to verify medical characteristics of the patient prior to delivery of a drug. The biosensors provide real-time data of drug levels and/or health related markers, offering valuable insights for personalized treatment for triggering by either the skin touch or the integrated sensor device. For example, a biosensor can monitor an opioid, the opioid agonist, or the partial opioid agonist, wherein the biosensor comprises a pulse oximeter, a heart rate sensor, an Electrocardiogram (ECG) sensor, a skin sensor, a temperature sensor, and/or a blood flow sensor.

The microprocessor in the AIMD handles data flow by acting as the central processing unit that coordinates all the AIMD functions. It receives input from various sensors, which monitor the internal environment and drug levels. The microprocessor processes this data to determine the appropriate actions, such as adjusting the dosage or triggering the actuator to release medication. It also manages communication with external devices through wireless modules, ensuring that data such as dosing history, sensor readings, and battery status are transmitted for remote monitoring and control. Additionally, the microprocessor stores critical data in its memory, including fault conditions and medical history, which can be accessed and analyzed to optimize treatment. By efficiently managing data flow, the microprocessor ensures that the device operates smoothly, making real-time adjustments based on sensor inputs and maintaining reliable communication with external systems. This integrated approach helps maintain the device's performance and enhances patient safety.

In an embodiment, the AIMD has a power supply module. The electronic module and the power supply module are separated from the rest of the modules of the device by a waterproofing. A power module of an implantable drug delivery device comprises at least one power source and a power switch. In some embodiments, the device harnesses energy (or power) from the environment through energy harvesting. The energy could be harvested by converting kinetic energy of body movements into electrical power, or by utilizing body heat to generate power, or by capturing energy from the heartbeat, or by using the body's electromagnetic fields. In some embodiments, the device receives power through induction charging from an external source. In some embodiments, the power switch is a magnetic field activated switch. The power switch can also utilize other mechanisms available in the environment to turn the power on permanently. The power source and the power switch work together to ensure the device operates reliably, utilizing available energy efficiently and maintaining a consistent power supply. The device relies on a wireless charger to recharge its rechargeable battery, ensuring a consistent supply of electric power.

**FIG. 2A** illustrates an AIMD comprising a check valve 220, according to one or more embodiments. The other components (such as drug chamber 202, drug 204, motor chamber 206, housing 208, motor 210, driving component 212, piston 214, electronic module 216, sensor module 217, one or more orifices 218), and the workings of the AIMD are already explained in FIG. 1. The AIMD may comprise a check valve 220 as shown in FIG. 2A. The check valve 220 is also known as a non-return valve. The check valve 220 is coupled to the drug delivery end of the AIMD. The check valve 220 is more specifically coupled to the AIMD via the one or more orifices 218. The check valve 220 is adapted to allow the fluid (liquid or gas) to flow in one direction while preventing reverse flow. The check valve 220 allows one-way flow of the drug from the drug chamber 202 to outside the AIMD through the one or more orifices 218. The check valve 220 blocks entry of a fluid to the drug chamber from outside the AIMD. The check valve 220 operates automatically, using a movable component like a disc, ball, or flap that opens under forward flow pressure and closes when flow reverses. This prevents backflow, protects equipment, contamination, and maintains system pressure. The check valve 220 remains closed until the incoming pressure when the drug releases reaches a threshold pressure.

The check valve 220 shown in FIG. 2A is a spring-loaded ball check valve. The spring-loaded ball check valve is a variation of the ball check valve. In this type of valve, a spring is added to the design to ensure quicker and more reliable sealing when the flow reverses. The primary components include a spherical ball, a spring, a seat for sealing, and a housing to contain the mechanism. The working principle involves two key stages. During forward flow, fluid pressure overcomes the spring's force and pushes the ball away from its seat, allowing fluid to flow through the spring-loaded ball check valve. The spring compresses as the ball moves, creating a clear pathway for the fluid. When the flow stops or reverses, the spring forces the ball back onto the seat, providing a tight seal that prevents backflow. This design ensures faster closure compared to standard ball check valves, making it ideal for systems prone to water hammer or sudden pressure changes. FIG. 2B depicts a schematic diagram of the check valve shown in FIG. 2A.

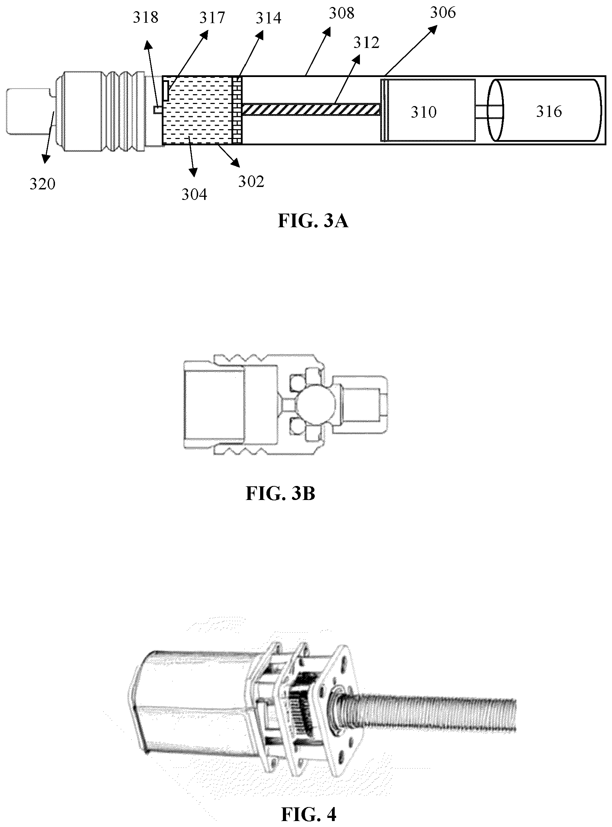

Similarly, **FIG. 3A** illustrates an AIMD comprising a check valve 320, according to one or more embodiments. The other components (such as drug chamber 302, drug 304, motor chamber 306, housing 308, motor 310, driving component 312, piston 314, electronic module 316, sensor module 317, one or more orifices 318) and the workings of the AIMD are already explained in FIG. 1. The check valve 320 shown in FIG. 3A is a ball check valve. The ball check valve is a type of check valve that uses a spherical ball to control the flow of fluid, allowing it to pass in one direction while preventing reverse flow. During forward flow, the fluid pressure pushes the ball away from its seat, creating a pathway for the fluid to pass. When reverse flow occurs, the pressure forces the ball back onto its seat, creating a tight seal that blocks the flow. The check valve 320 operates automatically based on pressure differences and does not require external controls, though some designs incorporate a spring to ensure quicker closure and prevent issues like water hammer. The check valve 320 remains closed until the incoming pressure reaches a threshold pressure.

The ball check valves are valued for their simple, compact design and minimal maintenance. **FIG. 3B** depicts a schematic diagram of the check valve shown in FIG. 3A.

**FIG. 4** illustrates a lead screw and motor arrangement, according to one or more embodiments. The lead screw is affixed to the motor shaft such that when the motor shaft rotates, the lead screw rotates. The lead screw may be affixed to the motor shaft through a gear mechanism. The gear mechanism may be compact to achieve compact size. The lead screw is a mechanical screw with helical threads. The helical threads may be located on the outer surface of the screw. The lead screw and motor arrangement may comprise a piston (not shown in FIG. 4). The piston comprises a hole in the centre. The piston also comprises threads on the inner surface of the hole in the piston. The threads in the piston match with the threads in the lead screw such that when the motor shaft rotates, the piston moves forward like the tool holder in a lathe. The movement of the piston pushes the drug (e.g., medication) through the valve.

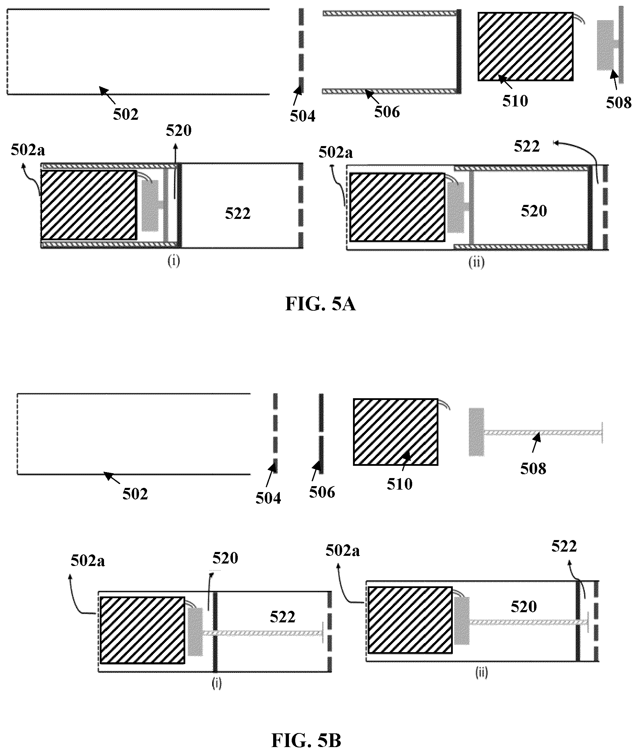

**FIG. 5A** illustrates an AIMD comprising a moving grooved cylinder on lead screw, according to one or more embodiments. Referring to **FIG. 5A****,** the external enclosure 502 is the main outer cylinder that has a first open end and a second perforated end 502a. The open end can accommodate the valve 504. The piston assembly 506 comprises a piston fixed to a separate cylinder that has an internal groove, similar to the thread of a nut. The motor and shaft assembly 508 comprises a motor that has a shaft end that has an outer groove, similar to the thread of a bolt or screw. When the motor shaft rotates, the grooved shaft interacts with the grooved cylinder that the piston is fixed to. This causes the piston assembly 506 to move forward and backward, similar to a lead screw mechanism. In some embodiments, the motor shaft is not able to move laterally. Instead, the rotating motion of the motor shaft pushes the cylinder that the piston is fixed to. This causes the piston to move forward. The movement of the piston pushes the medication 522 through the valve 504 at the front. The space created on the motor side of the piston is filled with interstitial fluid 520 coming through the perforated holes in the side wall of the external enclosure 502. The interstitial fluid 520 filling the space behind the piston helps maintain the pressure and prevent a vacuum from forming, which could otherwise cause the system to collapse. The electronics unit 510 comprises a power source comprising a battery and electronic components.

In some embodiments, the battery and electronic components are housed separately, likely in a smaller inner cylinder. This allows the main piston assembly to move without the need to accommodate the electronics within the same space.

An embodiment relates to a device comprising an external enclosure, a valve, a piston assembly, a motor and shaft assembly, an electronics unit, and a control switch.

**FIG. 5B** illustrates an AIMD comprising a moving piston on lead screw, according to one or more embodiments. Referring to **FIG. 5B****,** the external enclosure 502 is the main outer cylinder that has a first open end and a second perforated end 502a. The open end can accommodate the valve 504. The piston assembly 506 comprises a piston with a hole fixed to a separate cylinder that has internal grooves, similar to the threads of a nut. The motor and shaft assembly 508 comprises a motor shaft. Whether it is a stepper motor or a Direct Current (DC) motor, it functions similar to a lead screw in a lathe, facilitating precise linear movement. The shaft passes through the hole in the piston. As the motor shaft rotates, it moves the piston. This motion mimics the forward movement of a tool holder in a lathe, effectively pushing the piston forward. As the piston advances, it expels medication 522 through the valve 504. Simultaneously, the space created on the motor side of the piston is replenished by interstitial fluid 520 that enters through holes in the side wall of the external enclosure 502, ensuring a continuous operation. The electronics unit 510 comprises a power source comprising a battery and electronic components.

An embodiment relates to a device comprising an external enclosure, a valve, a piston assembly, a motor, and shaft assembly comprising a gear, an electronics unit, and a control switch.

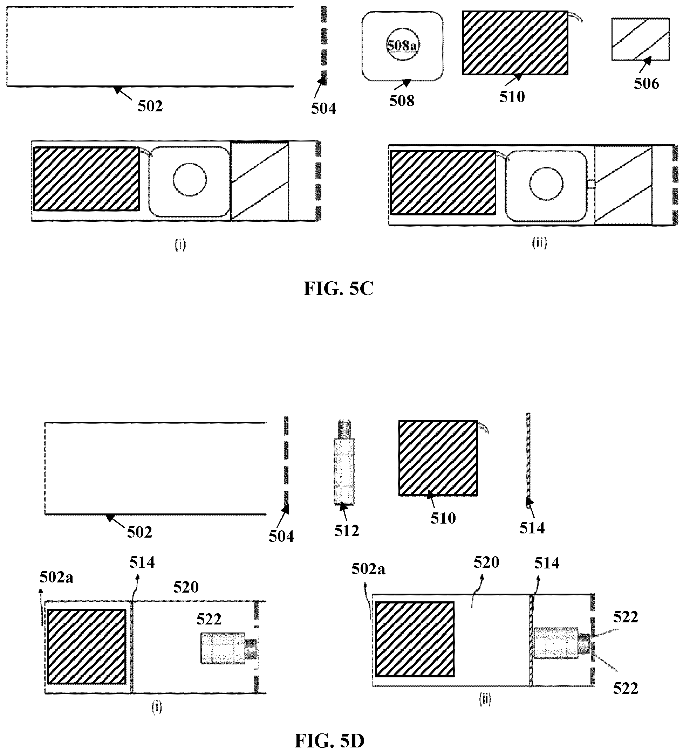

**FIG. 5C** illustrates an AIMD comprising a moving cylinder using gears, according to one or more embodiments. Referring to **FIG. 5C****,** the external enclosure 502 comprises a main outer cylinder with a first open end and a second perforated end, where the open end accommodates the valve. The valve 504 is positioned at the open end of the external enclosure. The piston assembly 506 includes a piston with a hole, fixed to a separate cylinder with internal grooves similar to nut threads. The motor and shaft assembly 508 features a motor shaft, either a stepper or DC motor, functioning like a lead screw in a lathe to facilitate precise linear movement. A gear 508a is attached to the motor shaft. The electronics unit 510 controls the motor and the overall device operation. The operation involves a gear mechanism where the gear attached to the motor shaft drives the motor shaft. As the gear rotates, it drives the motor shaft, which passes through the hole in the piston, mimicking the forward movement of a tool holder in a lathe and pushing the piston forward. This movement expels medication through the valve 504. The space created on the motor side of the piston is replenished by interstitial fluid 520 entering through holes in the side wall of the external enclosure 502, ensuring continuous operation. This design simplifies the device using a gear to drive the motor shaft, which in turn moves the piston, ensuring precise and continuous medication delivery.

An embodiment relates to a device comprising an external enclosure, a valve, a submersible pump, an electronics unit, and a control switch. The submersible pump sucks in the fluid and pushes it out.

**FIG. 5D** illustrates an AIMD comprising a submersible pump, according to one or more embodiments. Referring to **FIG. 5D****,** the external enclosure 502 of the device is designed as a main outer cylinder with a first open end and a second perforated end 502a. At the open end, a valve 504 is strategically positioned to facilitate the expulsion of medication D. Inside the enclosure, a submersible pump assembly 512 is placed near the perforated end, where the pump is responsible for sucking in the medication 522 and pushing it out. This submersible pump assembly 512 comprises either a stepper motor or a DC motor, which ensures precise medication 522 movement and effective operation. An integrated electronics unit 510 manages the motor and overall device functionality, allowing for accurate control of the pump's operation. During the pump's mechanism, the motor drives the submersible pump to draw medication 522 in through the perforated end, subsequently pushing it toward the valve 504 at the open end. As the pump operates, the medication is expelled through the valve 504, delivering the necessary medication 522. To maintain continuous operation, any space created by the expelled fluid is replenished by interstitial fluid 520 entering through the perforated end. A sliding spacer 514 maintains a barrier between the drug and the interstitial fluid.

This design not only simplifies the system by eliminating the need for complex piston and gear mechanisms but also enhances fluid handling efficiency, ensuring smooth and reliable medication 522 delivery. The compact nature of the submersible pump assembly 512 further makes this device suitable for a range of medical applications, requiring efficiency and reliability in delivering precise medication. A sliding spacer 514 maintains a barrier between the interstitial fluid and the drug reservoir.

An embodiment relates to a device comprising an external enclosure, a compressible drug chamber, a valve, a submersible pump, electronics unit, and a control switch. The submersible pump sucks in the fluid and pushes it out.

**FIG. 5E** illustrates an AIMD comprising a submersible pump and a compressible drug chamber, according to one or more embodiments. Referring to **FIG. 5E****,** the external enclosure 502 of the device is designed as a main outer cylinder with a first open end and a second perforated end 502a. At the open end, a valve 504 is strategically positioned to facilitate the expulsion of medication. Inside the enclosure, a submersible pump assembly 512 is placed near the perforated end, where the pump is responsible for sucking in medication 522 and pushing it out. The submersible pump assembly 512 is driven by either a stepper motor or a DC motor, which ensures precise drug movement and effective operation. An integrated electronics unit 510 manages the motor and overall device functionality, allowing for accurate control of the pump's operation. During the pump's mechanism, the motor drives the submersible pump assembly 512 to draw medication 522 in through the perforated end, subsequently pushing it toward the valve 504 at the open end. As the submersible pump assembly 512 operates, the drug is expelled through the valve 504, delivering the necessary medication. To maintain continuous operation, any space created by the expelled fluid is replenished by interstitial fluid 520 entering through the perforated end. Due to the pressure exerted by the interstitial fluid and removal of the drug from the compressible drug chamber 518 compresses gradually.

This design not only simplifies the system by eliminating the need for complex piston and gear mechanisms but also enhances drug dosing efficiency, ensuring smooth and reliable medication delivery. The compact nature of the submersible pump further makes this device suitable for a range of medical applications, requiring efficiency and reliability in delivering precise medication.

**FIG. 6A** illustrates an AIMD comprising a permeability module 622 on a motor chamber 606, according to one or more embodiments. The AIMD in FIG. 6A is similar to the one shown in FIG. 1. The other components (such as drug chamber 602, drug 604, motor chamber 606, housing 608, motor 610, driving component 612, piston 614, electronic module 616, sensor module 617, one or more orifices 618, check valve 620) of the AIMD are already explained in FIG. 1. The AIMD shown in FIG. 6A comprises the permeability module 622 on the housing (outer surface) of the motor chamber 606 in addition to the AIMD shown in FIG. 1. The housing of the motor chamber 606 comprises the permeability module 622. The permeability module 622 functions to seal the interior of the AIMD from the external environment, allowing only specific fluid molecules to permeate through a membrane plug into the device's interior. In an embodiment, permeability module 622 also effectively prevents items within the AIMD, such as an osmotic agent and a drug 604, from passing through it.

In an embodiment, the first end of the permeability module 622 has a semipermeable membrane (or membrane plug) supported by a plate, and the other end of the permeability module 622 has holes to allow inflow of fluid inside the device. In an embodiment, the permeability module 622 may have a hollow fiber or bundles of hollow fibers arranged together, forming a bridge for entry of outside fluid into the device. In an embodiment, permeability module 622 may also be designed to separate solutes from a feed solution, such as blood serum, using a semipermeable membrane.

In an embodiment, permeability module 622 has a permeability membrane held by a support structure as shown in **FIG. 6B****.** In an embodiment, support structure or plate could be made of a biocompatible material and/or metal such as without limitation titanium, Food and Drug Administration (FDA) approved material such as stainless steel. In some embodiments, the semipermeable membrane is supported by one plate or sandwiched between two rigid plates. These plates have a plurality of holes in front and back of the semipermeable membrane. The arrangement of these holes determines the flow rate of fluid passing through them. (Liquid or fluid are interchangeably used throughout the description.)

In an embodiment, a fluid ingress flow can be adjusted by adjusting the alignment of the rigid discs. When the rigid discs are aligned differently, the flow rate changes. When the rigid discs are aligned such that none of the holes from the first plate overlay the holes in the second plate, the flow rate through the sandwich structure is nearly zero. Conversely, when the rigid discs are aligned such that all the holes in the first plate overlay the holes in the second plate, the flow rate through the sandwich structure is maximum. By aligning the rigid discs so that partial areas of the holes in the first plate overlay the holes in the second plate, the flow rate falls between nearly zero and the maximum. This intermediate configuration allows fine-tuning of the ingress flow rate. In some embodiments, ingress flow rate of fluids through the permeability module could be adjusted by changing the membrane's permeability or its thickness.

The permeability module may comprise a hollow fiber membrane. As shown in **FIG. 6C****,** a hollow fiber membrane 624 comprises numerous thin, flexible fibers 626 bundled together in a casing 628. Each fiber has a hollow core, allowing fluid to flow through. Both ends of the fibers on the right side and the left side of the fibers are U shaped.

In one embodiment, the hollow fiber membrane is a forward osmosis membrane and comprises an inlet end facing the first end of the device and an outlet facing towards a sensor module. These membranes utilize the natural osmosis process, where fluid moves from a low concentration to a high concentration through the membrane. Natural osmosis requires lower energy compared to traditional reverse osmosis systems that operate under lower pressure conditions within hollow fibers.

In an embodiment, one or more hollow fibers are made up of semipermeable membranes. In an embodiment, the hollow fibers may be surrounded by a high concentrated glucose solution (G) as shown in the figure. The high concentration of the glucose solution allows natural osmosis to happen within the device. As fluid ingress flows from the body into the permeability module, an almost equal volume of the glucose solution will be pushed out from the permeability module through the opening at the second end (630) of the module. The hollow fiber forward osmosis membranes are thin and flexible.

The permeability module 622 may comprise a semipermeable membrane and an osmotic agent chamber. The permeability module 622 allows ingress flow of fluid to an osmotic agent chamber and generates osmotic pressure. The fluid may be a bodily fluid. The fluid may enter the osmotic agent chamber from outside the AIMD through the semipermeable membrane. The osmotic pressure is generated in response to the ingress flow of the fluid. The osmotic pressure drives the piston to move longitudinally towards the one or more orifices in addition to the actuation of the motor. The osmotic pressure acts as an additional force. The osmotic pressure is directly proportionate to the movement of the piston longitudinally. The ingress flow of the fluid may also trigger the actuation of the motor. In such a case, the ingress flow of the fluid may control the discharge of the drug.

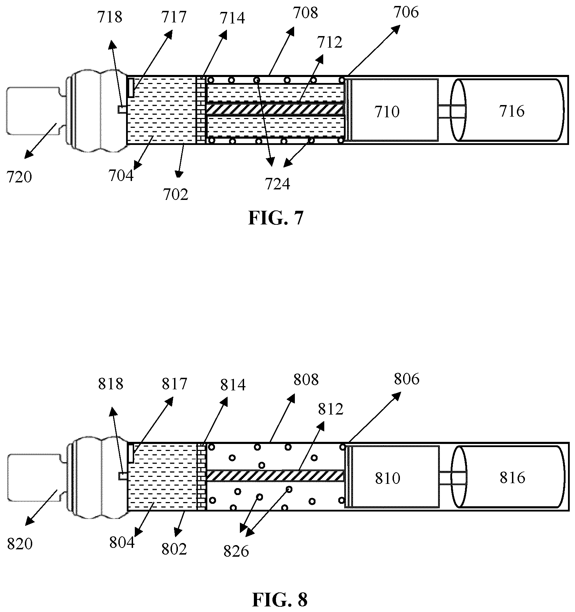

**FIG. 7** illustrates an AIMD comprising one or more provisions on a motor chamber to allow flow of interstitial fluid and prevent creation of vacuum, according to one or more embodiments. The AIMD in FIG. 7 is similar to the one shown in FIG. 1. The other components (such as drug chamber 702, drug 704, motor chamber 706, housing 708, motor 710, driving component 712, piston 714, electronic module 716, sensor module 717, one or more orifices 718, check valve 720) of the AIMD are already explained in FIG. 1. The AIMD shown in FIG. 7 comprises the one or more provisions 724 on the housing (outer surface) of the motor chamber 706 in addition to the AIMD shown in FIG. 1. The housing of the motor chamber 706 comprises the one or more provisions 724. The housing of the motor chamber 706 may also comprise filters along with the one or more provisions 724. The one or more provisions 724 may be holes.

**FIG. 8** illustrates an AIMD that pressurizes gas 826 on a motor chamber to prevent creation of vacuum, according to one or more embodiments. The other components (such as drug chamber 802, drug 804, motor chamber 806, housing 808, motor 810, driving component 812, piston 814, electronic module 816, sensor module 817, one or more orifices 818, check valve 820) of the AIMD are already explained in FIG. 1. The displacement of piston within the AIMD may lead to creation of vacuum which needs to be prevented. The creation of vacuum within the AIMD incurs risks such as cavitation risks, pressure differential risks, biocompatibility risks, outgassing risks, etc. If a vacuum environment is inadvertently created or leaks inside a sealed chamber within the AIMD, it could lead to cavitation risks such as structural failures or implosions. The vacuum inside the device can create pressure differential risks such as pressure imbalances that might lead to damage or reduced functionality. The vacuum can also lead to biocompatibility risks such as affecting the encapsulating materials or causing microcracks, potentially leading to exposure of internal components to the body. The vacuum may also interfere with pressure inside the AIMD that affects the operation of certain components. Further any residual vacuum in materials could release trapped gases over time, potentially interfering with AIMD performance or causing localized tissue reactions. The AIMD is designed in such a way to prevent creation of vacuum within the AIMD.

The electronic module may trigger opening a gas storage chamber (not shown) based on a signal received from the sensor module. The sensor module upon monitoring the displacement of the piston may communicate the signal to open the gas storage chamber. The gas storage chamber may be affixed externally to the AIMD. The gas storage chamber may comprise a valve that introduces the gas 826 into the AIMD. The pressure of the gas 826 may be varied (e.g., increased or decreased) by adjusting the regulator of the valve. The AIMD may use microvalves or elastomeric membranes to adjust internal pressure dynamically. The gas 826 may expand and occupy the area that is created due to the displacement of the piston. The gas 826 expands and restricts the creation of vacuum within the AIMD.

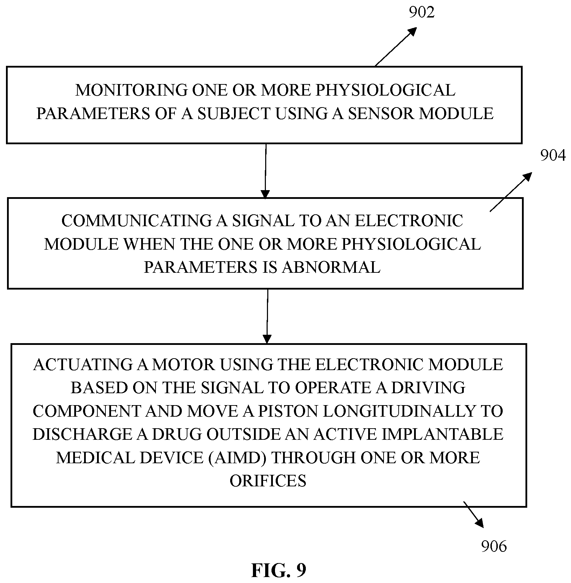

**FIG. 9** illustrates a method of actuating a motor and discharging a drug, according to one or more embodiments. The method comprises the following technical steps. At step 902, one or more physiological parameters of a subject are monitored using a sensor module. At step 904, a signal is communicated to an electronic module when the one or more physiological parameters is abnormal. At step 906, a motor is actuated using the electronic module based on the signal to operate a driving component and move a piston longitudinally to discharge a drug to a subject outside an active implantable medical device (AIMD) through one or more orifices.

The one or more physiological parameters are monitored continuously. The sensor module may be affixed to appropriate regions onto the subject. The sensor module may be placed within the subject. The sensor module may be communicatively coupled to the AIMD. The sensor communicates the signal to an electronic module when the one or more physiological parameters is abnormal. The electronic module actuates the motor. The motor upon actuation operates a driving component and moves a piston longitudinally to discharge a drug to a subject outside an active implantable medical device (AIMD) through one or more orifices.

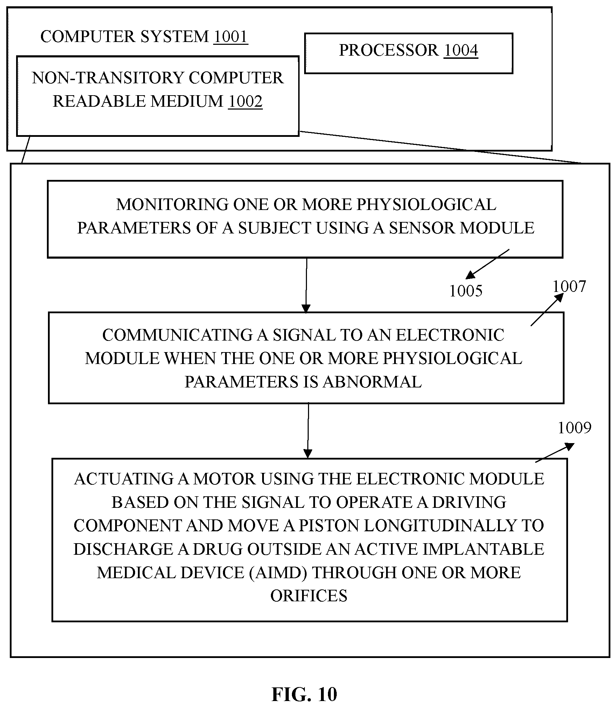

**FIG. 10** illustrates a non-transitory computer readable storage medium, according to one or more embodiments. According to an embodiment, disclosed is a computer system 1001 comprising the non-transitory computer readable storage medium 1002 having stored thereon instructions executable by a processor 1004 to perform operations comprising: monitoring one or more physiological parameters of a subject using a sensor module (at step 1005); communicating a signal to an electronic module when the one or more physiological parameters is abnormal (at step 1007); and actuating a motor using the electronic module based on the signal to operate a driving component and move a piston longitudinally to discharge a drug to the outside of an active implantable medical device (AIMD) through one or more orifices (at step 1009). The sensor module may be placed within the subject. The sensor module may be communicatively coupled to the AIMD.

An active implantable medical device (AIMD) is described herein. The AIMD comprises: a drug chamber that comprises a drug; a motor chamber that comprises a housing, a motor, and a driving component; a piston that is affixed to a first end of the driving component; and an electronic module that actuates the motor to operate the driving component and move the piston longitudinally to discharge the drug outside the AIMD through one or more orifices.

**Industrial Applicability:** The industrial applicability of the active implantable medical device (AIMD) lies in its potential use in the medical device industry, particularly in the fields of drug delivery systems and implantable medical devices. This AIMD can be used to provide precise, controlled, and long-term drug administration for patients with chronic conditions or requiring sustained treatment, offering significant benefits for personalized medicine. The device's application is suitable for both human healthcare and veterinary medicine, allowing for customized drug release, which could improve patient outcomes and enhance compliance by reducing the need for manual intervention. Additionally, this technology could be utilized in biopharmaceutical manufacturing for developing devices that ensure accurate and efficient drug dosing for various therapeutic areas, including pain management, hormone replacement, cancer treatment, and other long-term therapies. Its miniaturized, automated drug delivery mechanism makes it especially relevant for industries focused on creating devices that are both compact and highly effective, meeting the growing demand for implantable and wearables in healthcare.

It should be noted that the flowchart and the suggested time limits and parameters are meant to be exemplary, and that there could be other measures or criteria used in order to maximize safety and accuracy.

In the following, a set of items is disclosed. The items are numbered to facilitate referencing the features of one item in other items. The items form part of the present disclosure and could be made subject to independent and/or dependent claims irrespective of what currently is claimed in the application and also independent of the references in brackets if there are any. We note, however, that the scope of protection is defined by the appended claims, where the following items do not constitute claims. The items are:
1. An active implantable medical device (AIMD), comprising:
   a drug chamber that comprises a drug;
   a motor chamber that comprises a housing, a motor, and a driving component;
   a piston that is affixed to a first end of the driving component; and
   an electronic module that actuates the motor to operate the driving component and move the piston longitudinally to discharge the drug outside the AIMD through one or more orifices.
2. The AIMD of item 1, wherein the driving component comprises a helical threaded screw.
3. The AIMD of any one of the preceding items, wherein the AIMD further comprises a check valve coupled to the one or more orifices.
4. The AIMD of item 3, wherein the check valve allows one-way flow of the drug from the drug chamber to outside the AIMD through the one or more orifices.
5. The AIMD of item 3 or 4, wherein the check valve blocks entry of a fluid to the drug chamber from outside the AIMD.
6. The AIMD of any one of the preceding items, wherein the piston and the driving component moves together longitudinally to discharge the drug upon actuating the motor.
7. The AIMD of any one of the preceding items, wherein the AIMD comprises a sensor module.
8. The AIMD of item 7, wherein the sensor module monitors one or more physiological parameters of a subject.
9. The AIMD of item 8, wherein the sensor module communicates a signal to the electronic module when the one or more physiological parameters of the subject is abnormal.
10. The AIMD of any one of items 7 to 9, wherein the sensor module communicates a signal to the electronic module at a prescribed time.
11. The AIMD of any one of the preceding items, e.g. item 1, 9 or 10, wherein the electronic module actuates the motor in response to a signal received from the sensor module.
12. The AIMD of any one of the preceding items, wherein an outer circumference of the piston comprises a sealing component.
13. The AIMD of item 12, wherein the sealing component prevents leakage of the drug from the drug chamber to the motor chamber.
14. The AIMD of item 12 or 13, wherein the sealing component prevents contamination of the drug in the drug chamber with a fluid or gas in the motor chamber.
15. The AIMD of any one of the preceding items, wherein the motor chamber is configured to pressurize a gas within the motor chamber of the AIMD.
16. The AIMD of item 15, wherein the pressurization of the gas restricts creation of vacuum within the AIMD when the piston moves longitudinally towards the one or more orifices.
17. The AIMD of item 16, wherein the gas is an inert gas.
18. The AIMD of any one of the preceding items, wherein the housing comprises a permeability module.
19. The AIMD of item 18, wherein the permeability module comprises a semipermeable membrane and an osmotic agent chamber.
20. The AIMD of item 18 or 19, wherein the permeability module allows ingress flow of a fluid to an osmotic agent chamber and generates osmotic pressure.
21. The AIMD of item 20, wherein the osmotic pressure drives the piston to move longitudinally towards the one or more orifices in addition to the actuation of the motor.
22. The AIMD of item 21, wherein the osmotic pressure is directly proportionate to the movement of the piston longitudinally.
23. The AIMD of any one of the preceding items 20 to 22, wherein the ingress flow of the fluid controls the discharge of the drug.
24. The AIMD any one of the preceding items, wherein the housing of the motor chamber comprises one or more provisions and a filter.
25. The AIMD of item 24, wherein the one or more provisions allows ingress or ingress flow of a fluid from outside the AIMD to the motor chamber through the filter.
26. The AIMD of item 25, wherein the ingress or ingress flow of the fluid from outside the AIMD to the motor chamber prevents creation of vacuum within the AIMD.
27. The AIMD of any one of the preceding items, wherein the electronic module comprises a microprocessor.
28. The AIMD of any one of the preceding items, wherein an outer circumference of the piston is in surface compliance with the AIMD.
29. The AIMD of any one of the preceding items, wherein a first length of the driving component is equal to sum of a second length of the motor, a third length between an initial position of the piston and a motor position, and a fourth length between the initial position of the piston and a displacement position of the piston.
30. An active implantable medical device (AIMD), comprising:
   a drug chamber that comprises a drug;
   a motor chamber that comprises a housing, a motor, and a driving component;
   a stationary guiding component comprising a provision through which the driving component protrudes longitudinally; and
   an electronic module that actuates the motor to operate the driving component and protrude longitudinally through the stationary guiding component to discharge the drug outside the AIMD through one or more orifices.
31. The AIMD of item 30, wherein the driving component comprises a flattened surface at a first end to push the drug through the one or more orifices.
32. The AIMD of item 30 or 31, wherein the AIMD comprises a sealing component between the stationary guiding component and the driving component.
33. The AIMD of any one of the preceding items 30 to 32, wherein the driving component is operable to function as a piston to discharge the drug outside the AIMD through the one or more orifices.
34. The AIMD of any one of the preceding items30 to 33, wherein the driving component comprises a helical threaded screw.
35. The AIMD of any one of the preceding items 30 to 34, wherein the AIMD further comprises a check valve coupled to the one or more orifices.
36. The AIMD of item 35, wherein the check valve allows one-way flow of the drug from the drug chamber to outside the AIMD through the one or more orifices.
37. The AIMD of item 35 or 36, wherein the check valve blocks entry of a fluid to the drug chamber from outside the AIMD.
38. The AIMD of any one of the preceding items 30 to 37, wherein the stationary guiding component comprises one or more bumps configured to prevent rotation of the stationary guiding component.
39. The AIMD of any one of the preceding items 30 to 38, wherein the AIMD comprises a sensor module.
40. The AIMD of item 39, wherein the sensor module monitors one or more physiological parameters of a subject.
41. The AIMD of item 40, wherein the sensor module communicates a signal to the electronic module when the one or more physiological parameters of the subject is abnormal.
42. The AIMD of any one of the preceding items 39 to 41, wherein the sensor module communicates a signal to the electronic module at a prescribed time.
43. The AIMD of item 41, wherein the electronic module actuates the motor in response to a signal received from the sensor module.
44. The AIMD of any one of the preceding items 30 to 43, wherein an outer circumference of the stationary guiding component comprises a sealing component.
45. The AIMD of item 44, wherein the sealing component prevents leakage of the drug from the drug chamber to the motor chamber.
46. The AIMD of item 45, wherein the sealing component prevents contamination of the drug in the drug chamber with a fluid or gas in the motor chamber.
47. The AIMD of any one of the preceding items 30 to 46, wherein the motor chamber is configured to pressurize a gas within the motor chamber of the AIMD.
48. The AIMD of item 47, wherein the pressurization of the gas restricts creation of vacuum within the AIMD when the driving component moves longitudinally towards the one or more orifices.
49. The AIMD of item 48, wherein the gas is an inert gas.
50. The AIMD of any one of the preceding items 30 to 49, wherein the housing comprises a permeability module.
51. The AIMD of item 50, wherein the permeability module comprises a semipermeable membrane and an osmotic agent chamber.
52. The AIMD of item 50 or 51, wherein the permeability module allows ingress flow of a fluid to an osmotic agent chamber and generates osmotic pressure.
53. The AIMD of item 52, wherein the osmotic pressure pushes the driving component to move longitudinally towards the one or more orifices in addition to the actuation of the motor.
54. The AIMD of item 52 or 53, wherein the osmotic pressure is directly proportionate to the movement of the driving component longitudinally.
55. The AIMD of any one of the preceding items 52 to 54, wherein the ingress flow of the fluid controls the discharge of the drug.
56. The AIMD of any one of the preceding items 30 to 55, wherein the housing of the motor chamber comprises one or more provisions and a filter.
57. The AIMD of item 56, wherein the one or more provisions allows ingress flow of a fluid from outside the AIMD to the motor chamber through the filter.
58. The AIMD of item 57, wherein the ingress flow of the fluid from outside the AIMD to the motor chamber prevents creation of vacuum within the AIMD.
59. The AIMD of any one of the preceding items 30 to 58, wherein the electronic module comprises a microprocessor.
60. The AIMD of item 31 or any one of the other preceding items 30 and 32 to 59 when additionally referring to item 31, wherein an outer circumference of the flattened surface is in surface compliance with the AIMD.
61. The AIMD of any one of the preceding items 30 to 60, e.g. item 31, wherein a first length of the driving component is equal to sum of a second length of the motor, a third length between an initial position of the first end of the driving component and a motor position, and a fourth length between the initial position of the first end of the driving component and a displacement position of the first end of the driving component.
62. A method comprising:
   monitoring one or more physiological parameters of a subject using a sensor module;
   communicating a signal to an electronic module when the one or more physiological parameters is abnormal; and
   actuating a motor using the electronic module based on the signal to operate a driving component and move a piston longitudinally to discharge a drug to a subject outside an active implantable medical device (AIMD) through one or more orifices.
63. The method of item 62, wherein the sensor module is placed within the subject.
64. The method of item 62 or 63, wherein the sensor module is communicatively coupled to the AIMD.
65. A non-transitory computer readable storage medium comprising a sequence of instructions, which when executed by a processor causes:
   monitoring one or more physiological parameters of a subject using a sensor module;
   communicating a signal to an electronic module when the one or more physiological parameters is abnormal; and
   actuating a motor using the electronic module based on the signal to operate a driving component and move a piston longitudinally to discharge a drug to a subject outside an active implantable medical device (AIMD) through one or more orifices.
66. The non-transitory computer readable storage medium of item 65, wherein the sensor module is placed within the subject.
67. The non-transitory computer readable storage medium of item 65 or 66, wherein the sensor module is communicatively coupled to the AIMD.

**In** the following, another set of items is disclosed. The items are numbered to facilitate referencing the features of one item in other items. The items form part of the present disclosure and could be made subject to independent and/or dependent claims irrespective of what currently is claimed in the application and also independent of the references in brackets if there are any. We note, however, that the scope of protection is defined by the appended claims, where the following items do not constitute claims. The items are:
1. An active implantable medical device (AIMD), comprising:
   a drug chamber that comprises a drug;
   a motor chamber that comprises a housing, a motor, and a driving component;
   a piston that is affixed to a first end of the driving component; and
   an electronic module that actuates the motor to operate the driving component and move the piston longitudinally to discharge the drug outside the AIMD through one or more orifices; and
   a permeability module that allows passive ingress flow of a fluid to an osmotic agent chamber from outside the AIMD through a semipermeable membrane and generates osmotic pressure to drive the piston longitudinally towards the one or more orifices in addition to actuation of the piston by the motor.
2. The AIMD of item 1, wherein the driving component comprises a helical threaded screw that links the piston to a motor shaft and converts rotational motion to linear motion and moves the piston longitudinally.
3. The AIMD of item 1, wherein the AIMD further comprises a check valve coupled to the one or more orifices to allow one-way flow of the drug from the drug chamber to outside the AIMD through the one or more orifices.
3. The AIMD of item 1, wherein the piston and the driving component move together longitudinally to discharge the drug upon actuating the motor.
4. The AIMD of item 1, wherein the AIMD comprises a sensor module configured to monitor one or more physiological parameters of a subject and communicate a signal to the electronic module when the one or more physiological parameters of the subject is abnormal.
5. The AIMD of item 4, wherein the electronic module actuates the motor in response to the signal received from the sensor module.
6. The AIMD of item 1, wherein the motor chamber is configured to pressurize a gas within the motor chamber of the AIMD.
7. The AIMD of item 6, wherein the pressurization of the gas restricts creation of vacuum within the AIMD when the piston moves longitudinally towards the one or more orifices.
8. The AIMD of item 1, wherein the permeability module is a forward osmosis membrane.
9. The AIMD of item 8, wherein a first end of the permeability module comprises the semipermeable membrane sandwiched between rigid plates.
10. The AIMD of item 9, wherein a second end of the permeability module permits the passive ingress flow of the fluid to an osmotic agent chamber of the AIMD.
11. The AIMD of item 1, wherein the housing of the motor chamber comprises one or more provisions and a filter.
12. The AIMD of item 11, wherein the one or more provisions allows ingress flow of a fluid from outside the AIMD to the motor chamber through the filter.
13. The AIMD of item 12, wherein the ingress flow of the fluid from outside the AIMD to the motor chamber prevents creation of vacuum within the AIMD.
14. The AIMD of item 1, wherein the electronic module comprises a microprocessor.
15. A method comprising:
   monitoring one or more physiological parameters of a subject using a sensor module;
   determining passive ingress flow of a fluid to an osmotic agent chamber from outside an active implantable medical device (AIMD) through a semipermeable membrane; communicating a signal to an electronic module when the one or more physiological parameters is abnormal; and
   actuating a motor using the electronic module based on the signal to operate a driving component and move a piston longitudinally to discharge a drug to a subject outside the active implantable medical device (AIMD) through one or more orifices.
16. The method of item 15, wherein the sensor module is placed within the subject.
17. The method of item 15, wherein the sensor module is communicatively coupled to the AIMD.
18. A non-transitory computer readable storage medium comprising a sequence of instructions, which when executed by a processor causes:
   monitoring one or more physiological parameters of a subject using a sensor module;
   determining passive ingress flow of a fluid to an osmotic agent chamber from outside an active implantable medical device (AIMD) through a semipermeable membrane;
      communicating a signal to an electronic module when the one or more physiological parameters is abnormal; and
      actuating a motor using the electronic module based on the signal to operate a driving component and move a piston longitudinally to discharge a drug to a subject outside the active implantable medical device (AIMD) through one or more orifices.
19. The non-transitory computer readable storage medium of item 18, wherein the sensor module is communicatively coupled to the AIMD.

### Reference Signs List:

102, 202, 302, 602, 702, 802 - Drug chamber
104, 204, 304, 604, 704, 804 - Drug
106, 206, 306, 606, 706, 806 - Motor chamber
108, 208, 308, 608, 708, 808 - Housing
110, 210, 310, 610, 710, 810 - Motor
112, 212, 312, 612, 712, 812 - Driving component
114, 214, 314, 614, 714, 814 - Piston
116, 216, 316, 616, 716, 816 - Electronic Module
117, 217, 317, 617, 717, 817 - Sensor Module
118, 218, 318, 618, 718, 818 - Orifice
220, 320, 620, 720, 820 - Check Valve
622 - Semipermeable membrane
724 - provisions
826 - gas
502 - external enclosure
502a - second perforated end
504 - valve
506 - piston assembly
508 - motor and shaft assembly
508a - gear
510 - electronics unit
512 - submersible pump assembly
514 - sliding spacer
518 - drug chamber
624 - hollow fiber membrane
626 - flexible fibers
628 - casing
630 - second end
902, 904, 906 - Method steps
1001 - Computer system
1002 - Non-transitory computer readable medium
1004 - processor
1005, 1007, 1009 -Steps

## Claims

1. An active implantable medical device (AIMD), comprising:
a drug chamber that comprises a drug;
a motor chamber that comprises a housing, a motor, and a driving component;
a piston that is affixed to a first end of the driving component; and
an electronic module that actuates the motor to operate the driving component and move the piston longitudinally to discharge the drug outside the AIMD through one or more orifices; and
a permeability module that allows passive ingress flow of a fluid to an osmotic agent chamber from outside the AIMD through a semipermeable membrane and generates osmotic pressure to drive the piston longitudinally towards the one or more orifices in addition to actuation of the piston by the motor.

2. The AIMD of claim 1, wherein the driving component comprises a helical threaded screw that links the piston to a motor shaft and converts rotational motion to linear motion and moves the piston longitudinally.

3. The AIMD of claim 1 or 2, wherein the AIMD further comprises a check valve coupled to the one or more orifices to allow one-way flow of the drug from the drug chamber to outside the AIMD through the one or more orifices.

4. The AIMD of any one of the preceding claims, wherein the piston and the driving component move together longitudinally to discharge the drug upon actuating the motor.

5. The AIMD of any one of the preceding claims, wherein the AIMD comprises a sensor module configured to monitor one or more physiological parameters of a subject and communicate a signal to the electronic module when the one or more physiological parameters of the subject is abnormal.

6. The AIMD of claim 5, wherein the electronic module actuates the motor in response to the signal received from the sensor module.

7. The AIMD of any one of the preceding claims, wherein the motor chamber is configured to pressurize a gas within the motor chamber of the AIMD, wherein, optionally, the pressurization of the gas restricts creation of vacuum within the AIMD when the piston moves longitudinally towards the one or more orifices.

8. The AIMD of any one of the preceding claims, wherein
a) the permeability module is a forward osmosis membrane, and/or
b) the housing of the motor chamber comprises one or more provisions and a filter, and/or
c) the electronic module comprises a microprocessor.

9. The AIMD of any one of the preceding claims, e.g. claim 1 or claim 8, option a), wherein a first end of the permeability module comprises the semipermeable membrane sandwiched between rigid plates, wherein, optionally, a second end of the permeability module permits the passive ingress flow of the fluid to an osmotic agent chamber of the AIMD.

10. The AIMD of claim 8, option b), wherein the one or more provisions allows ingress flow of a fluid from outside the AIMD to the motor chamber through the filter, wherein, optionally, the ingress flow of the fluid from outside the AIMD to the motor chamber prevents creation of vacuum within the AIMD.

11. A method comprising:
monitoring one or more physiological parameters of a subject using a sensor module;
determining passive ingress flow of a fluid to an osmotic agent chamber from outside an active implantable medical device (AIMD) through a semipermeable membrane; communicating a signal to an electronic module when the one or more physiological parameters is abnormal; and
actuating a motor using the electronic module based on the signal to operate a driving component and move a piston longitudinally to discharge a drug to a subject outside the active implantable medical device (AIMD) through one or more orifices.

12. The method of claim 11, wherein the sensor module is placed within the subject.

13. The method of claim 11 or 12, wherein the sensor module is communicatively coupled to the AIMD.

14. A non-transitory computer readable storage medium comprising a sequence of instructions, which when executed by a processor causes:
monitoring one or more physiological parameters of a subject using a sensor module;
determining passive ingress flow of a fluid to an osmotic agent chamber from outside an active implantable medical device (AIMD) through a semipermeable membrane;
communicating a signal to an electronic module when the one or more physiological parameters is abnormal; and
actuating a motor using the electronic module based on the signal to operate a driving component and move a piston longitudinally to discharge a drug to a subject outside the active implantable medical device (AIMD) through one or more orifices.

15. The non-transitory computer readable storage medium of claim 14, wherein the sensor module is communicatively coupled to the AIMD.
